(19) Europäisches Patentamt
European Patent Office
Office européen des brevets

(11) Veröffentlichungsnummer: **0 039 001**
**B1**

(12) **EUROPÄISCHE PATENTSCHRIFT**

(45) Veröffentlichungstag der Patentschrift: **08.06.83**

(51) Int. Cl.³: **C 07 C 19/02**, C 07 C 17/16

(21) Anmeldenummer: **81102901.6**

(22) Anmeldetag: **15.04.81**

(54) **Verfahren zur Herstellung von Methylchlorid.**

(30) Priorität: **26.04.80 DE 3016220**

(43) Veröffentlichungstag der Anmeldung: **04.11.81 Patentblatt 81/44**

(45) Bekanntmachung des Hinweises auf die Patenterteilung: **08.06.83 Patentblatt 83/23**

(84) Benannte Vertragsstaaten: **BE DE FR GB IT NL**

(56) Entgegenhaltungen:
**DE-B-1 907 088**
**DE-B-2 031 000**

(73) Patentinhaber: **HOECHST AKTIENGESELLSCHAFT, Postfach 80 03 20, D-6230 Frankfurt/Main 80 (DE)**

(72) Erfinder: **Grünbein, Wolfgang, Dr., Alt-Oberliederbach 35, D-6237 Liederbach (DE)**
Erfinder: **Lendle, Wilhelm, Dr., Hirschpfad 5, D-6232 Bad Soden am Taunus (DE)**
Erfinder: **Post, Hendrik Willem, Paulinenweg 4, D-6238 Hofheim am Taunus (DE)**
Erfinder: **Richter, Heinz, Wilhelm-Bonn-Strasse 2, D-6242 Kronberg/Taunus (DE)**
Erfinder: **Rossberg, Manfred, Dr., Am Rotfeld 8, D-6273 Waldems (DE)**

**EP 0 039 001 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Methylchlorid

Die Erfindung betrifft ein Verfahren zur Herstellung von Methylchlorid durch Umsetzung von Methanol mit Chlorwasserstoff in der Gasphase an einem Aluminiumoxid-Katalysator bei erhöhter Temperatur und einem Druck von mindestens 1 bar.

Derartige Verfahren sind bereits bekannt, z.B. aus US-PS 1 834 089, US-PS 1 920 246, Faith et al.: Industrial Chemicals (1957) 513–516, DE-AS 1 907 088 und Schlosser et al.: Chemie-Ingenieur-Technik 42 (1970), 1215–1219. Bei diesen Verfahren werden Chlorwasserstoff und Methanol gasförmig und vorgewärmt einem Reaktor zugeführt, in dem die exotherme Umsetzung an dem Katalysator nach der Gleichung

$$CH_3OH + HCl \rightarrow CH_3Cl + H_2O$$

erfolgt.

Der Nachteil der bekannten Verfahren besteht darin, dass der Katalysator relativ schnell inaktiv wird. Dieser Vorgang wird von einer Kohlenstoff-Abscheidung begleitet. Die Inaktivierung zeigt sich darin, dass am Reaktorausgang steigende Mengen an nicht umgesetztem Methanol anfallen.

Wesentlich beschleunigt wird die Inaktivierung, wenn mehrfach chlorierte $C_1$- und $C_2$-Kohlenwasserstoffe, wie Tetrachlorkohlenstoff, Chloroform oder Tetrachlorethan bei der Umsetzung zugegen sind. Derartige Stoffe sind häufig in technischem Chlorwasserstoff enthalten, der als Nebenprodukt bei anderen Verfahren, z.B. Chlorierungen anfällt.

Der Erfindung liegt die Aufgabe zugrunde, die Geschwindigkeit der Inaktivierung und die Kohlenstoff-Abscheidung zu vermindern.

Gegenstand der Erfindung ist ein Verfahren zur Herstellung von Methylchlorid durch Umsetzung von Methanol mit Chlorwasserstoff in der Gasphase an einem Aluminiumoxid-Katalysator bei erhöhter Temperatur und einem Druck von mindestens 1 bar, das dadurch gekennzeichnet ist, dass man die Umsetzung in Gegenwart von 1 bis 80 l Sauerstoff, bezogen auf 20°C und 1 bar, pro kg eingesetztes Methanol durchführt.

Vorzugsweise beträgt die Sauerstoffmenge (bezogen auf 20°C und 1 bar) 1 bis 50 l, insbesondere 1 bis 30 l, jeweils pro kg eingesetztes Methanol.

Der Sauerstoff kann in reiner Form oder in Form eines sauerstoffhaltigen Gases, insbesondere als Luft eingesetzt werden, und zwar kontinuierlich oder diskontinuierlich.

Die Umsetzung kann in einem Reaktionsrohr durchgeführt werden, das mit dem Katalysator gefüllt ist. Vorzugsweise benutzt man einen Reaktor, der aus einer Vielzahl von solchen Rohren besteht, die in einem Bündel angeordnet sind und von aussen gekühlt werden. Zur Kühlung wird das Rohrbündel zweckmässig in einen Mantel eingeschlossen, durch den ein Kühlmedium fliesst.

Bei der Umsetzung stellt sich in der in den Rohren befindlichen Katalysatorschüttung ein Temperaturprofil ein, d.h. die Temperatur steigt hinter dem Reaktoreingang zunächst auf ein Maximum und fällt dann in Richtung des Reaktorausgangs – bedingt durch die Kühlung – wieder ab. Die Temperatur in der Katalysatorschüttung stellt man dabei über die Kühlung im allgemeinen auf Werte zwischen 250 und 500°C, vorzugsweise zwischen 300 und 420°C ein.

Die Umsetzung wird bei einem Druck von mindestens 1 bar, vorzugsweise bei 1 bis 11 bar, insbesondere bei 1 bis 5 bar durchgeführt.

Der eingesetzte Aluminiumoxid-Katalysator hat vorzugsweise eine spezifische Oberfläche von 120 bis 200 $m^2/g$ und kann körnig sein oder aus geformten Teilchen bestehen. Geeignete Katalysatorformen sind z.B. Kugeln oder Zylinder.

Das Molverhältnis von Methanol zu Chlorwasserstoff beträgt vorzugsweise 0.75:1 bis 1:1.

Falls man für die Umsetzung Chlorwasserstoff einsetzen will, der mehrfach chlorierte $C_1$- und $C_2$-Kohlenwasserstoffe enthält, so empfiehlt es sich, darauf zu achten, dass die Konzentration dieser Verbindungen Werte unter 5 Vol.%, vorzugsweise unter 2 Vol.% hat, jeweils bezogen auf das gesamte Einsatzgas. Dies lässt sich durch Kühlung des Chlorwasserstoffs erreichen, wobei die chlorierten Kohlenwasserstoffe mehr oder weniger weitgehend – je nach Intensität der Kühlung – auskondensieren.

Vergleichsbeispiel

Ein Gasstrom bestehend aus 6,9 mol/h HCl, 5,8 mol/h $CH_3OH$, sowie 0,29 mol/h $CCl_4$ wird an einem handelsüblichen Aluminiumoxid-Katalysator umgesetzt, der eine spezifische Oberfläche von 200 $m^2/g$ hat. Der Katalysator befindet sich in einem Nickelrohr von 50 mm Durchmesser, das durch einen Ofen von aussen beheizt wird. Die Länge der Katalysator-Schüttung in diesem Rohr beträgt 400 mm, die maximale Temperatur des Katalysators wurde mit 420°C gemessen. Der Überschuss an HCl und die Reaktionsprodukte verlassen den Reaktor gasförmig.

Bereits nach 52,5 h beginnt die Selektivität der Reaktion

$$CH_3OH + HCl \rightarrow CH_3Cl + H_2O$$

von 94.5% stark abzufallen, und zwar auf 23.5% nach weiteren 64.5 Stunden. Nach Beendigung des Versuchs lassen sich grosse Mengen Kohlenstoff nachweisen, die in dem Reaktor abgeschieden wurden.

Beispiel

Wird einem Gasstrom von 6,9 mol/h HCl, 5,7 mol/h $CH_3OH$ sowie 0,29 mol/h $CCl_4$ Sauerstoff in einer Menge von 0,17 mol/h zugesetzt,

unter sonst gleichen Bedingungen wie im Vergleichsbeispiel, so wird auch nach 150 Stunden kein wesentlicher Abfall der Selektivität beobachtet. Die Kohlenstoff-Abscheidung war gegenüber dem Vergleichsbeispiel um den Faktor 14 verringert.

## Patentansprüche

1. Verfahren zur Herstellung von Methylchlorid durch Umsetzung von Methanol mit Chlorwasserstoff in der Gasphase an einem Aluminiumoxid-Katalysator bei erhöhter Temperatur und einem Druck von mindestens 1 bar, dadurch gekennzeichnet, dass man die Umsetzung in Gegenwart von 1 bis 80 l Sauerstoff, bezogen auf 20°C und 1 bar, pro kg eingesetztes Methanol durchführt.

2. Verfahren nach Anspruch 1, dadurch gekennzeichnet, dass die Sauerstoffmenge 1 bis 50 l pro kg Methanol beträgt.

3. Verfahren nach Anspruch 1, dadurch gekennzeichnet, dass die Sauerstoffmenge 1 bis 30 l pro kg Methanol beträgt.

## Revendications

1. Procédé de préparation du chlorure de méthyle par réaction du méthanol avec le chlorure d'hydrogène en phase gazeuse, en présence d'un catalyseur d'oxyde d'aluminium, à température élevée et sous une pression d'au moins 1 bar, procédé caractérisé en ce que la réaction est effectuée en présence de 1 à 80 litres d'oxygène (volume ramené à 20°C et à 1 bar) par kg de méthanol mis en jeu.

2. Procédé selon la revendication 1, caractérisé en ce que la quantité d'oxygène utilisée est de 1 à 50 litres par kg de méthanol.

3. Procédé selon la revendication 1, caractérisé en ce que la quantité d'oxygène utilisée est de 1 à 30 litres par kg de méthanol.

## Claims

1. A process for the manufacture of methyl chloride by reaction of methanol with hydrogen chloride in the gaseous phase, in the presence of an aluminium oxide catalist, at elevated temperature and under a pressure of at least 1 bar, characterized by carrying out the reaction in the presence of 1 to 80 liters of oxygen, relative to 20°C and 1 bar, per kg of methanol used.

2. The process as claimed in claim 1, wherein the amount of oxygen is from 1 to 50 liters per kg of methanol.

3. The process as claimed in claim 1, wherein the amount of oxygen is from 1 to 30 liters per kg of methanol.